# EUROPEAN PATENT APPLICATION

(11) **EP 3 919 087 A1**
(43) Date of publication of application: **08.12.2021**
(21) Application number: 21177610.9
(22) Date of filing: 03.06.2021
(51) Int. Cl.: A61L 9/12, C04B 33/04, C04B 35/14, C04B 38/00

(54) **PROCESS FOR MAKING A ROD-LIKE CERAMIC DIFFUSER AND ROD-LIKE CERAMIC DIFFUSER FOR DIFFUSING A VOLATILE SUBSTANCE**

(30) Priority: 03.06.2020 IT 202000013084
(71) Applicant: Opera IP Ltd., London W14 9NL (GB)
(72) Inventor: FADELLI, Celso, London (GB)
(74) Representative: Gallo, Luca

(57) **Abstract**

Process for making a rod-like ceramic diffuser for diffusing a volatile substance, which comprises a step of arranging a paste mixture constituted by a solvent and by ceramic powders dispersed in the solvent. In particular, the solvent is present in the mixture in a quantity lower than 40 % by weight with respect to the weight of the mixture.

The process also comprises an extrusion step, in which the paste mixture is forced to pass through a shaped die with the formation of a continuous semifinished product with elongated shape.

In addition, the process comprises a step of firing the semifinished product, in which the solvent is removed and the semifinished product is sintered in order to obtain a rod-like ceramic diffuser. In particular, the firing step heats the semifinished product to a firing temperature comprised between 900 and 1100 °C.

## Description

### Field of application

The present invention regards a process for making a rod-like ceramic diffuser and a rod-like ceramic diffuser for diffusing a volatile substance according to the preamble of the main independent claims.

The present process is generally inserted in the industrial field of production of diffusers of volatile substances, in particular of ambient fragrance dispensers.

The diffuser, advantageously attained by means of the aforesaid process, is inserted in the field of home articles, in particular intended for decorative use and for scenting environments or for the diffusion or essences, or for the diffusion of insect repellents. The process is therefore advantageously intended to be used for producing rod-like ceramic diffusers with different shapes, sizes and use destinations.

### State of the art

In the field of home articles intended for scenting environments, rod-like diffusers are known that are made of fibrous material, e.g. bamboo or another type of wood, which are provided with an elongated body intended to be partially immersed with one end in a scented liquid (hereinbelow also simply indicated as "liquid") within a container.

Due to the particular fibrous structure of the diffuser, the liquid rises from the immersed end at its interior, generally via capillarity, up to reaching an opposite end of the diffuser placed outside the container.

The liquid thus tends to be uniformly distributed over the entire volume of the diffuser up to reaching the external surface of the diffuser itself, where it evaporates and is diffused in the external environment. Such process occurs with a continuous supply of new liquid on the external surface of the diffuser as the liquid itself progressively evaporates due to the capillarity of the material. Through the entire lateral surface of the diffuser, therefore, there is the diffusion of the essence into the air of the surrounding environment. The process is stopped when the liquid in the container has run out.

In particular, in order to obtain a scented liquid that is not excessively volatile, the latter generally comprises a mixture of water, alcohol and fragrance.

Indeed, a correct balancing between water and alcohol allows obtaining a rather high volatility (due to the volatility of the alcohol) to allow a considerable diffusion in the environment, but also a volatility sufficiently low (due to the volatility of the water) to allow a prolonged use of the diffuser without having to supply new liquid in the container.

The above-described diffusers have in practice shown that they do not lack drawbacks.

A first drawback of the known diffusers lies in the fact that the material with which they are made easily deteriorates over time. Indeed, the wooden diffusers, even if not very costly, tend to be ruined if constantly soaked with liquid, to be swollen and worn out in a relatively quick manner and therefore they must be changed often.

Otherwise, if the wooden diffuser is treated with a varnish or another chemical product in order to be made more resistant and durable, at the end of its useful lifetime it would be difficult to recycle, compost or would not be biodegradable like untreated wood. In addition, the varnish would close the interstices between the fibers, negatively affecting the capillarity of the diffuser.

A further drawback of the known wooden diffusers lies in the fact that they cannot be reused, since their fibrous structure tends to retain a residue of the fragrance at its interior when the liquid is finished. Therefore, a fragrance that is subsequently used would be irreparably modified by the residue of the previous fragrance that remained in the diffuser. In addition, wooden diffusers are hard to wash, making it impossible to eliminate the aforesaid fragrance residue.

A further drawback of the known wooden diffusers lies in the fact that they are inflammable and therefore not particularly recommended for use in public places, for example in hotel rooms, in which they would represent a danger for safety, since they can be easily confused with incense burners.

A further drawback of the known wooden diffusers lies in the fact that, still due to their inflammability, they cannot be used in applications in which their heating is required, such as for example in devices for the diffusion of insect repellants. In such devices, in fact, the diffuser is heated by means of electrical heating elements, in order to consequently transfer heat to the absorbed liquid and increase the volatility thereof.

In order to find a solution to the drawbacks of the abovementioned diffusers, it is known to make the rod-like diffusers of ceramic material in place of wood.

It is known to make the ceramic diffusers via casting or injection of a substantially liquid or semi-liquid ceramic mixture (in jargon termed "slurry") within a mold. The mixture is subsequently left to dry in order to obtain a semifinished product (in jargon termed "raw"), which is subsequently sintered, i.e. fired at high temperatures, in order to acquire the typical mechanical strength of ceramic materials.

In this case, during use, the scented liquid in which the ceramic diffuser is immersed rises towards the external end to the container, always via capillarity, due to the porous structure of the ceramic material itself.

In order to prevent the closure of the surface pores of the ceramic material (and hence in order to not prevent the evaporation of the liquid), the latter is not glazed and refired. For example, a rod-like ceramic diffuser of known type, indicated above, is described in the document CN 1234639. In particular, such document describes a process for making a ceramic diffuser by means of injection in a mold of a semi-liquid ceramic mixture, which comprises clay, quartz and feldspar.

The semifinished product thus obtained is left to dry and is sintered at a temperature up to 1250 °C.

Also such rod-like ceramic diffusers have in practice shown that they do not lack drawbacks.

The main drawback of the abovementioned diffusers lies in the fact that they require long production times, mainly due to the drying time for the material within the mold and after the extraction. Indeed, in order to obtain a ceramic mixture that can be easily cast or injected with a good quality for filling the mold, it is necessary to add a quantity of solvent that is sufficiently high, which however must be removed during the drying in a slow manner so to not generate splits or breakage in the material.

A further drawback of the above-described ceramic diffusers lies in the fact that the pores of the material are not present in a sufficient quantity and they are not sufficiently connected to ensure a good capillarity. Indeed, the sintering process decreases the dimensions of the pores, with the increase of the temperature at which it is executed, until a percentage of the pores themselves has disappeared. The high temperature are also responsible for possible deformations and breakage due to the heat shrinkage and internal tensions caused thereby.

A further drawback of the above-described ceramic diffusers lies in the fact that in order to confer to the semifinished product a sufficient strength for being extracted from the mold, the ceramic mixture must comprise a sufficiently high quantity of organic binder, which is removed via high-temperature calcination during the sintering of the semifinished product. In particular, the calcination of the organic binder produces gases which can increase the pores of the material, but can also generate cracks that compromise the mechanical strength thereof. Indeed, in the event in which the gas produced during calcination is unable to reach the external environment, i.e. if the pores are not interconnected, the internal pressure of the gas generates internal tensions on the ceramic material. The outflow of the gas is however obstructed by the high sintering temperature which reduces the dimensions of the pores, as indicated above.

A further drawback of the above-described ceramic diffusers lies in the fact that they cannot be provided with a section with particularly complex shape, since the mold cannot be provided with undercuts. In addition, the shape must be sufficiently simple to facilitate the extraction of the semifinished product from the mold itself.

The documents WO 2009/073813 and ES 2303455 describe other processes of known type for making ceramic diffusers by means of the extrusion of a paste mixture comprising ceramic powders.

The documents JP 08084766 and IT MI20112282 describe further processes of known type for making ceramic diffusers by means of molding of ceramic materials with clay or terracotta base.

Also the latter solutions of known type non do not lack drawbacks tied in particular to the high use of solvent and of organic binders.

### Presentation of the invention

In this situation, the problem underlying the present invention is therefore that of overcoming the drawbacks shown by the abovementioned solutions of known type by providing a process for making a rod-like ceramic diffuser which requires a lower use of solvent and of organic binders.

A further object of the present invention is to provide a rod-like ceramic diffuser which has an improved capillarity with respect to the known diffusers.

A further object of the present invention is to provide a process which allows obtaining rod-like ceramic diffusers with sections having shape that is even complex.

A further object of the present invention is to provide a rod-like ceramic diffuser which is strong and durable over time.

A further object of the present invention is to provide a rod-like ceramic diffuser which is reusable.

A further object of the present invention is to provide a process and a rod-like ceramic diffuser which are simple and inexpensive to make.

### Brief description of the drawings

The technical characteristics of the present invention, according to the aforesaid objects, can be seen in the contents of the below-reported claims and the advantages thereof will be more evident in the following detailed description, made with reference to the enclosed figures, which represent a merely exemplifying and non-limiting embodiment of the invention in which:
- figure 1 shows a perspective view of an assembly for diffusing a volatile substance, which comprises a rod-like ceramic diffuser according to the invention;
- figure 2 shows a perspective view of a rod-like ceramic diffuser in accordance with a preferred embodiment of the present invention;
- figure 3 shows a view from a different perspective of a detail of the rod-like ceramic diffuser of figure 2;
- figure 4 shows a section of the rod-like ceramic diffuser in accordance with a different embodiment of the present invention;
- figures 5a,b show two micrographs with different enlargements, obtained by means of scanning electron microscope, of a section of an embodiment of the invention, in which the pores of the rod-like ceramic diffuser are underlined with a darker tonality;
- figures 6a,b show two micrographs with different enlargements, obtained by means of scanning electron microscope, of the lateral surface of an embodiment of the invention, in which the pores of the rod-like ceramic diffuser are underlined with a darker tonality.

### Detailed description of several preferred embodiments

With reference to the enclosed drawings, reference number 1 overall indicates a rod-like ceramic diffuser 1 for diffusing a volatile substance.

With the expression "volatile substance" it is intended a substance (or a mixture of substances) provided with high vapor pressure, advantageously intended to pass from the liquid state L to the gaseous state at atmospheric temperature and pressure, for example a fragrance, an essence, an insect repellent.

According to the invention, the rod-like ceramic diffuser 1 is provided with an elongated body 10 extended mainly along a longitudinal direction Y between a first end 11, intended to be at least partially immersed in a liquid L comprising a volatile substance, and a free second end 12.

Advantageously, the elongated body 10 of the rod-like ceramic diffuser 1 is provided with uniform section along the longitudinal direction Y.

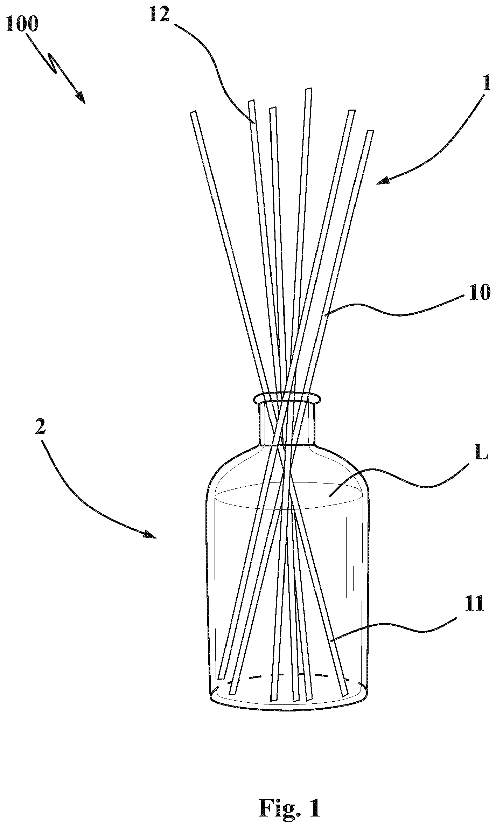

Advantageously, the ceramic diffuser 1 according to the invention is intended to be used in an assembly 100 for the diffusion of a volatile substance in an internal or external environment, as is for example illustrated in figure 1.

For example, the assembly 100 comprises a containment body 2, on which an opening is made and within which the liquid L is contained which comprises the volatile substance, e.g. a scented liquid L comprising a mixture of water, alcohol and a fragrance. The assembly 100 also advantageously comprises at least one rod-like ceramic diffuser 1 with the first end 11 immersed in the liquid L and the second end 12 placed externally with respect to the containment body 2.

Of course, without departing from the protective scope of the invention, the first end 11 and the second end 12 can be reversed, i.e. the second end 12 can be immersed in the liquid L, while the first end 11 can be outside the containment body 2. Advantageously, based on the size of the assembly 100 in which the diffuser is used, the elongated body 10 of the rod-like ceramic diffuser 1 is provided with a length between the first end 11 and the second end 12 comprised between 10 and 100 cm. For example, the elongated body 10 can be 15 cm long for assemblies in which the containment body 2 has a capacity of about 100 ml and over 40 cm for large-size containers.

In addition, the elongated body 10 is provided with section with average diameter comprised between 0.4 and 2 cm, preferably comprised between 0.6 and 1 cm.

In accordance with the idea underlying the present invention, the rod-like ceramic diffuser 1 is made of sintered ceramic material provided with an open porosity greater than 25 %, preferably comprised between 35 and 50 %.

With the expression "open porosity" it is intended the volumetric fraction of space occupied by the interconnected pores, i.e. in fluid communication with each other, i.e. pores which contribute to the capillarity of the rod-like ceramic diffuser 1. Advantageously, the rod-like ceramic diffuser 1 is made of sintered ceramic material obtained from a mixture of a solvent, preferably water, and ceramic powders, which is extruded and fired at a temperature lower than 1200°C, preferably at a temperature comprised between 900 and 1100 °C, and still more preferably between 980 and 1020 °C.

Advantageously, moreover, the aforesaid ceramic powders comprise calcium carbonate in a quantity greater than 20% by weight with respect to the weight of the ceramic powders themselves.

Advantageously, the high open porosity of the ceramic material allows obtaining a rod-like ceramic diffuser 1 with optimal capillarity, capable of making the liquid L rise along the elongated body 10 from the immersed first end 11 to the free second end 12. Advantageously, moreover, the addition of calcium carbonate allows lowering the sintering temperature and increasing the volume occupied by the open pores.

In this manner, it is possible to obtain a ceramic diffuser provided with high porosity and simultaneously with high mechanical properties.

Indeed, the use of high temperatures for the step of firing (sintering) the paste mixture, in addition to reducing the pores (therefore reducing the amount of liquid absorbed by the material), also cause deformations and breakage of the ceramic diffuser, in particular due to the thermal shrinkage and to the internal tensions.

Therefore, a high quantity of calcium carbonate within the ceramic powders allows obtaining a final product which, being sintered at lower temperatures, has smaller shrinkage and deformation phenomena associated therewith and is therefore provided with improved mechanical properties.

In addition, the paste mixture, object of the present invention, comprises ceramic powders whose composition, described in detail hereinbelow, also allows increasing the mechanical properties of the (raw) semifinished product before the firing step.

The improved mechanical properties obtained due to the aforesaid composition of the ceramic powders allow reducing the amount of (polymeric) organic binder necessary in the mixture.

Normally, the organic binder is in fact employed for conferred sufficient strength to the semifinished product such that it can be processed before the firing step.

Nevertheless the aforesaid organic binder is subsequently removed by calcination at high temperature during the sintering of the semifinished product, which can generate internal tensions and cracks which compromise the mechanical strength of the finished product.

More in detail, in the event in which the gas produced during calcination is unable to reach the outside environment, for example due to non-interconnected pores, the internal pressure of the gas generates internal tensions on the ceramic material which can also crack the material itself.

The outflow of the gas is also obstructed by a high sintering temperature which, as described above, reduces the dimensions of the pores.

Therefore, the paste mixture, object of the present invention, comprises ceramic powders whose composition allows obtaining a ceramic diffuser provided with improved mechanical properties.

More in detail, the aforesaid composition allows reducing the amount of organic binder employed and, by reducing the sintering temperature, simultaneously allows the gas produced during calcination to exit more easily from the mixture, reducing the risk of cracks and splits.

In order to further improve the capillarity, the rod-like ceramic diffuser 1 is also advantageously provided with pores having average diameter lower than 100 µm preferably lower than 50 µm.

Advantageously, the elongated body 10 of the rod-like ceramic diffuser 1 is provided with a shaped external surface 13, which is in fluid communication with the internal pores. In operation, the liquid L rises via capillarity along the elongated body 10 from the immersed first end 11 towards the free second end 12, being distributed over the entire volume, and in particular on the external surface 13, on which it evaporates. Therefore, the greater the external surface 13 of the elongated body 10, given the same mass of the elongated body 10 itself, the greater the quantity of volatile substance that is situated in contact with the environment and therefore can evaporate.

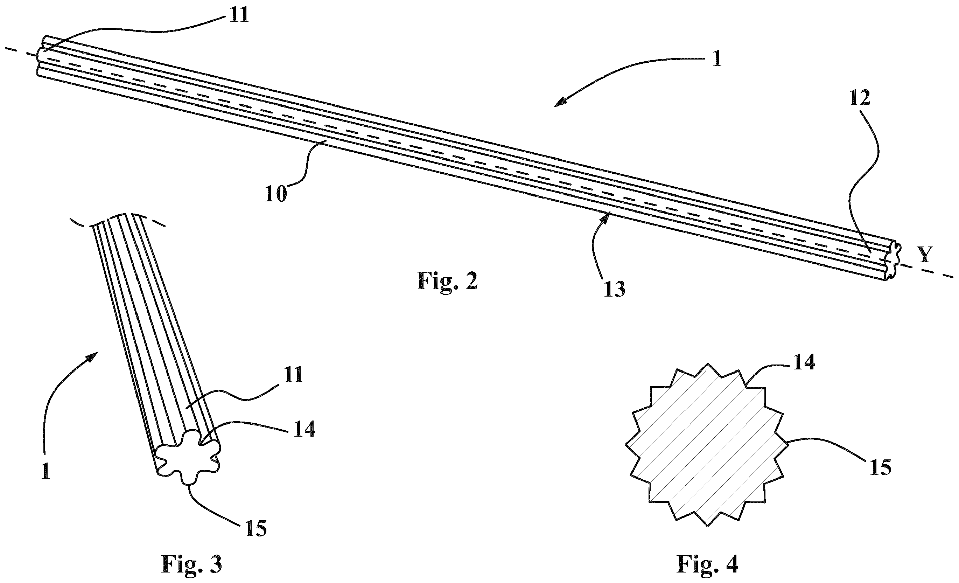

Therefore, the rod-like ceramic diffuser 1 is preferably provided with a specific external surface area greater than 0.3 cm²/g, preferably greater than 1 cm²/g. Advantageously, a high specific surface area is obtained by means of shaping the elongated body 10 so as to obtain a section with an edge provided with concave sections 14 and convex sections 15, i.e. a section with a high ratio between perimeter and surface, as in the two embodiment variants illustrated in the enclosed figures 2- 4.

Advantageously, the rod-like ceramic diffuser 1 is provided with a geometric density lower than 5 g/cm³, preferably lower than 2.5 g/cm³.

With the expression "geometric density" it is intended the density calculated by considering as volume the entire volume of the elongated body 10, i.e. the volume delimited by the external surface 13 of the elongated body 10. Such geometric density is distinguished from the "apparent density" due to the fact that the latter considers the volume without the open pores, and from the "real density" due to the fact that the latter considers the volume without all the pores present. A low geometric density therefore indicates advantageously a high volume of pores.

In accordance with the preferred embodiment of the present invention, the rod-like ceramic diffuser 1 is attained by means of extrusion of the mixture of solvent and ceramic powders. In order to maintain a sufficiently viscous mixture, the solvent is advantageously present in the mixture in a quantity lower than 40 % by weight with respect to the weight of the mixture.

The ceramic powders comprise calcium carbonate, quartz and clay.

More in detail, the ceramic powders comprise a quantity of calcium carbonate comprised between 20 and 35% by weight with respect to the weight of the ceramic powders, preferably between 25 and 30% by weight.

In particular, the calcium carbonate is advantageously in the form of marble powder, preferably dolomite.

In addition, the ceramic powders comprise a quantity of clay comprised between 35 and 55% by weight with respect to the weight of the ceramic powders, preferably between 40 and 50% by weight.

Advantageously, the clay comprises kaolin and is in particular preferably constituted by kaolin and quartz.

In addition, the ceramic powders comprise a quantity of quartz (not including possible quartz present in the clay) comprised between 15 and 30% by weight with respect to the weight of the ceramic powders, preferably between 20 and 25% by weight.

For example, the ceramic powders have soft earthenware base. With the expression "soft earthenware" it is intended a mixture, known in the field, in which the ceramic powders comprise various proportions of clay (50 to 60%), of quartz (28 to 40%) and of feldspar (8 to 12%), with the addition of limestone (up to reaching mass percentages comprised in the previously indicated intervals), i.e. calcium carbonate, which lowers the firing point.

In accordance with the preferred embodiment of the present invention, the rod-like ceramic diffuser 1 is provided with a water absorption capacity greater than 15%, preferably greater than 20%.

Also forming the object of the invention is a process for making the aforesaid rod-like ceramic diffuser 1, regarding which the same reference numbers will be maintained hereinbelow for the sake of description simplicity.

In accordance with the idea underlying the present invention, the process comprises a step of arranging a paste mixture comprising a solvent, preferably water and ceramic powders dispersed in the solvent.

With the term "paste" it is intended a semi-solid mixture with high viscosity, provided with a degree of saturation of the pores (DPS) of the solvent equal to about 1 or slightly higher.

Therefore, the solvent is present in the mixture in a quantity lower than 40 % by weight with respect to the weight of the mixture.

According to the invention, the process comprises an extrusion step, in which the paste mixture is forced to pass through a shaped die with the formation of a continuous semifinished product with elongated shape. In particular, the forming by means of extrusion advantageously allows decreasing the quantity of solvent used. Advantageously, the shaped die is provided with a shaped passage hole with a perimeter edge provided with a plurality of concave sections 14 alternated with convex sections 15, so as to obtain a continuous semifinished product (and a corresponding rod-like ceramic diffuser 1) with an external surface 13 with large extension.

Of course, without departing from the protective scope of the invention, the shaped die can have a simple shape, e.g. circular or square.

According to the invention, the process also comprises a step of firing the semifinished product, in which the solvent is removed and the semifinished product is sintered in order to obtain a rod-like ceramic diffuser 1.

In particular, the firing step heats the semifinished product to a firing temperature comprised between 900 and 1100 °C, preferably between 980 and 1020 °C. Advantageously, the process also comprises a drying step, preceding said firing step, in which a first portion of said solvent is removed, said drying step being executed in air preferably at a temperature lower than 100 °C.

Advantageously, the process comprises a cutting step, following the extrusion step and preceding the firing step, in which the continuous semifinished product is cut to size, advantageously keeping under consideration the possible shrinkage in the firing step. Of course, without departing from the protective scope of the present invention, the cutting step can also be executed following the firing.

By way of example, data is reported hereinbelow relative to one embodiment of a rod-like ceramic diffuser 1 obtained by following the present method and using the materials indicated in Table 1.

In accordance with the embodiment, the mixture is extruded, cut and dried in air. Subsequently, the semifinished product thus obtained is sintered to a firing temperature of 1000 °C.

**Table 1 - Materials used in order to obtain a ceramic rod-like diffuser**

| **Material** | **% by weight** |
|---|---|
| Ceramic powder: | 80-85 (over the total) |
| Kaolinite clay (kaolinite + quartz) | 40-50 |
| Quartz | 20-25 |
| Dolomite | 25-30 |
| Solvent (water) | 15-20 (over the total) |
| Additives (fluidifying) | < 0.4 (over the total) |

The rod-like ceramic diffuser 1 according to the embodiment is provided with an open porosity of 48.8% with a geometric density of 1.59 g/cm³. In particular, the percentage of open porosity was calculated by multiplying by 100 the difference between the value one and the ratio between the geometric density of the sample and the apparent density, which was measured by means of a helium pycnometer.

In particular, the sample is provided with an apparent density of 2.82 g/cm³ and a real density (of only the material without pores) of 2.85 g/cm³. Since the two porosity values are very similar, the porosity of the sample is nearly entirely open (percentage of closed pores of about 0.6%), allowing an optimal capillarity.

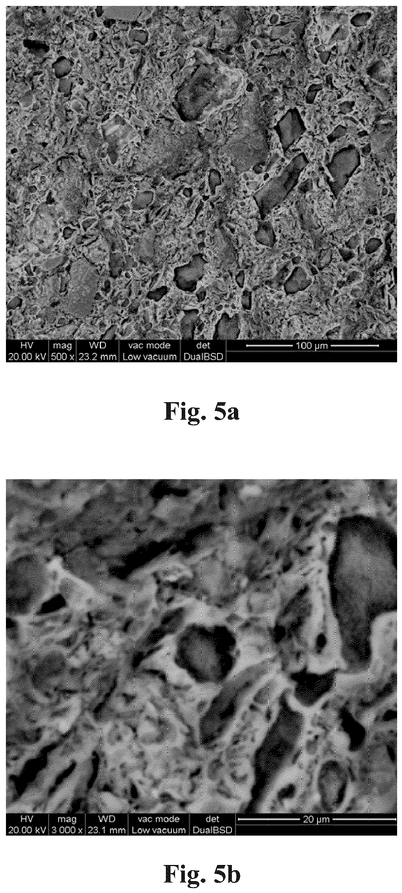

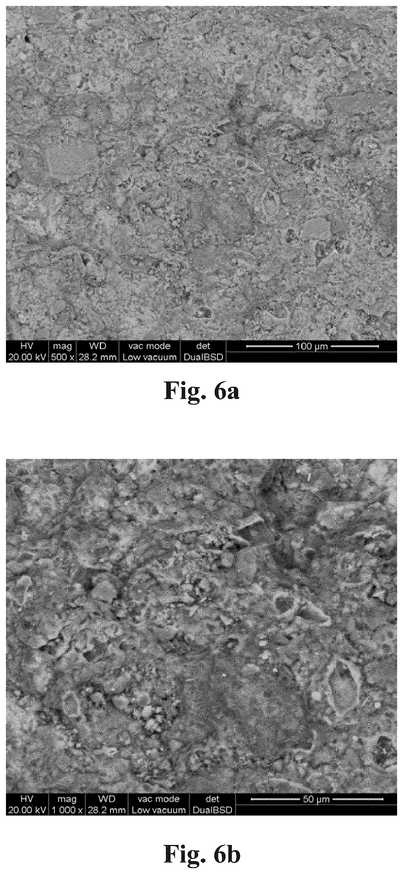

In addition, as shown in figures 5a,b and 6a,b, which illustrate morphological images of the sample obtained with a scanning electron microscope (SEM) with different enlargements, the average diameter of nearly all the pores is lower than 50 µm.

It is also possible to observe that, except for several larger pores, the dimensions of the pores are substantially uniform.

In addition, the rod-like ceramic diffuser 1 has a good absorption percentage, equal to 25%.

The invention thus conceived therefore attains the pre-established objects.

## Claims

1. Process for making a rod-like ceramic diffuser for diffusing a volatile substance, **characterized in that** it comprises the following steps:
- a step of arranging a paste mixture comprising:
- a solvent, and
- ceramic powders dispersed in said solvent, said ceramic powders comprising:
- calcium carbonate, in a quantity comprised between 25 and 30% by weight with respect to the weight of said ceramic powders,
- clay, in a quantity comprised between 35 and 55% by weight with respect to the weight of said ceramic powders, and
- quartz, in a quantity comprised between 15 and 30% by weight with respect to the weight of said ceramic powders;
said solvent being present in said mixture in a quantity lower than 40 % by weight with respect to the weight of the mixture;
- an extrusion step, in which said paste mixture is forced to pass through a shaped die with the formation of a continuous semifinished product with elongated shape;
- a step of firing said semifinished product, in which said solvent is removed and said semifinished product is sintered in order to obtain a rod-like ceramic diffuser (1); said firing step heating said semifinished product to a firing temperature comprised between 900 and 1100 °C.

2. Process according to claim 1, **characterized in that**, in said ceramic powders,
- said clay is present in a quantity comprised between 40 and 50% by weight with respect to the weight of said ceramic powders, and
- said quartz is present in a quantity comprised between 20 and 25% by weight with respect to the weight of said ceramic powders.

3. Process according to claim 2, **characterized in that**:
- said solvent is present in said mixture in a quantity comprised between 15 and 20 % by weight with respect to the weight of said mixture;
- said paste mixture comprises additives in a quantity smaller than 0.4 % by weight with respect to the total weight of said mixture.

4. Process according to any one of the preceding claims, **characterized in that** said calcium carbonate is in the form of marble powder, preferably dolomite.

5. Process according to any one of the preceding claims, **characterized in that** in said extrusion step said paste mixture is forced to pass through a shaped die with the formation of a continuous semifinished product with elongated shape;
said shaped die being provided with a shaped passage hole with a perimeter edge provided with a plurality of concave sections (14) alternated with convex sections (15).

6. Process according to any one of the preceding claims, **characterized in that** it comprises a drying step, preceding said firing step, in which a first portion of said solvent is removed, said drying step being executed in air.

7. Process according to any one of the preceding claims, **characterized in that** it comprises a cutting step, following said extrusion step, in which said continuous semifinished product is cut to size.

8. Rod-like ceramic diffuser for diffusing a volatile substance, attained by means of the process according to any one of the claims 1 to 7, which is **characterized in that** it is provided with an elongated body (10) extended mainly along a longitudinal direction (Y) between a first end (11), intended to be at least partially immersed in a liquid (L) comprising a volatile substance, and a free second end (12),
said rod-like ceramic diffuser (1) being made of sintered ceramic material provided with an open porosity greater than 25%.

9. Rod-like ceramic diffuser according to claim 8, **characterized in that** it is provided with pores having average diameter lower than 100 µm.

10. Rod-like ceramic diffuser according to claim 8 or 9, **characterized in that** it is provided with a specific external surface greater than 0.3 cm²/g.

11. Rod-like ceramic diffuser according to claim 10, **characterized in that** said elongated body (10) is provided with a section with an edge provided with concave sections (14) and convex sections (15).

12. Rod-like ceramic diffuser according to any one of the claims 8 to 11, **characterized in that** it is provided with a geometric density lower than 5 g/cm³.
